# EUROPEAN PATENT SPECIFICATION

(11) **EP 2 737 877 B1**
(45) Date of publication and mention of the grant of the patent: **03.02.2016**
(21) Application number: 14150741.8
(22) Date of filing: 17.10.2013
(51) Int. Cl.: A61F 2/46

(54) **Orthopaedic prosthesis assembly tool**
Orthopädisches Prothesen-Montagewerkzeug
Outil d'assemblage de prothèse orthopédique

(30) Priority: 18.10.2012 US 201213655015
(43) Date of publication of application: 04.06.2014
(62) Divisional of application: 13189135.0
(73) Proprietor: DePuy Synthes Products, LLC, Raynham, MA 02767-0350 (US)
(72) Inventor: McAnelly, Jeffrey A, Warsaw, IN Indiana 46581 (US); Satterthwaite, Rodney E, Warsaw, IN Indiana 46581 (US); Lashure, Daniel E, Warsaw, IN Indiana 46581 (US)
(74) Representative: Belcher, Simon James

(56) References cited:
- EP-A1- 2 397 111
- DE-U1-202012 102 017
- US-A1- 2007 123 908
- US-B1- 6 238 435

## Description

The present invention relates instruments for use in the assembly of orthopaedic joint prostheses.

Patients who suffer from the pain and immobility caused by osteoarthritis and rheumatoid arthritis have an option of joint replacement surgery. Joint replacement surgery is quite common and enables many individuals to function properly when it would not be otherwise possible to do so. Artificial joints are usually comprised of metal, ceramic and/or plastic components that are fixed to existing bone.

Such joint replacement surgery is otherwise known as joint arthroplasty. Joint arthroplasty is a well-known surgical procedure by which a diseased and/or damaged joint is replaced with a prosthetic joint. In a typical total joint arthroplasty, the ends or distal portions of the bones adjacent to the joint are resected or a portion of the distal part of the bone is removed and the artificial joint is secured thereto.

Many designs and methods for manufacturing implantable articles, such as bone prostheses, are known. Such bone prostheses include components of artificial joints such as elbows, hips, knees and shoulders.

During performance of a joint replacement procedure, it is generally necessary to provide the surgeon with a certain degree of flexibility in the selection of a prosthesis. In particular, the anatomy of the bone into which the prosthesis is to be implanted may vary somewhat from patient to patient. Such variations may be due to, for example, the patient's age, size and gender. For example, in the case of a femoral prosthesis, the patient's femur may be relatively long or relatively short thereby requiring use of a femoral prosthesis which includes a stem that is relatively long or short, respectively. Moreover, in certain cases, such as when use of a relatively long stem length is required, the stem must also be bowed in order to conform to the anatomy of the patient's femoral canal.

Such a need for prostheses of varying shapes and sizes thus creates a number of problems in regard to the use of a one-piece prosthesis. For example, a hospital must maintain a relatively large inventory of prostheses in order to have an appropriate selection of prostheses needed for certain situations, such as trauma situations and revision surgery. Moreover, since the bow of the stem must conform to the bow of the intramedullary canal of the patient's femur, rotational positioning of the upper portion of the prosthesis is limited, thereby rendering precise location of the upper portion and hence the head of the prosthesis very difficult. In addition, since corresponding bones of the left and right side of a patient's anatomy (e.g. left and right femur) may bow in opposite directions, it is necessary to provide variations of the prosthesis in order to provide anteversion of the bone stem, thereby further increasing the inventory of prostheses which must be maintained.

As a result of these and other drawbacks, a number of modular prostheses have been designed. As its name implies, a modular prosthesis is constructed in modular form so that the individual elements or figures of the prosthesis can be selected to fit the needs of a given patient's anatomy. For example, modular prostheses have been designed which include a proximal neck component which can be assembled to any one of numerous distal stem components in order to create an assembly which fits the needs of a given patient's anatomy. Such a design allows the distal stem component to be selected and thereafter implanted in the patient's bone in a position which conforms to the patient's anatomy while also allowing for a limited degree of independent positioning of the proximal neck component relative to the patient's pelvis.

One issue that arises as a result of the use of a modular prosthesis is the locking of the components relative to one another. In particular, firm reproducible locking of the proximal neck component to the distal stem component is critical to prevent separation of the two components subsequent to implantation thereof into the patient. The need for the firm locking is particularly necessary if the design does not provide for positive locking with weight bearing. As such, a number of locking mechanisms have been designed to lock the components of a modular prosthesis to one another. For example, a number of modular prostheses have heretofore been designed to include a distal stem component which has an upwardly extending post which is received into a bore defined distal neck component. A relatively long fastener such as a screw or bolt is used to secure the post in the bore. Other techniques of securing modular components include the impacting of one component onto the other. This technique can have variable results.

Current designs of modular stems include designs in which the modular connection utilizes a tapered fit between the two components. For example, the proximal body may include an internal taper which mates with an external taper on the distal stem. Such a taper connection may be used in conjunction with additional securing means, for example, a threaded connection or may be used alone. It is important that the tapered connection be secure. For example, the proper amount of force must be applied to the tapered connection to properly secure the tapered connection so that the connection can withstand the forces associated with the operation of the stem.

Current attempts to provide a device to adjoin components of a modular joint prosthesis are fraught with several problems. For example, the device may not provide sufficient mechanical advantage to securely lock the components. Further, the ergonomics available to lock the components may not be optimal. There is thus a need to provide for an assembly tool capable of alleviating at least some of the aforementioned problems.

EP-A-2397111 discloses an orthopaedic assembly tool for assembly of a first and second components of an orthopaedic prosthesis for use in joint arthroplasty. The tool includes a first member for engaging the first component and defining a first member longitudinal axis, and a second member for engaging the second component. The second member includes a tensile bar which is adapted to break at a predetermined force, and a housing adapted to retain the tensile bar after the tensile bar breaks,

The invention provides an assembly tool for assembly of a first component of a prosthesis to a second component of the prosthesis for use in joint arthroplasty, as defined in claim 1.

Optionally, the tensile bar is sized and shaped to break at a predetermined force of between about 8900 N and about 11100 N (about 2000 lbf and about 2500 lbf).

Optionally, the lower restraint includes a spring-loaded lower section adapted to contain the tensile bar in the lower restraint.

Optionally, in which the second member includes a cap having a threaded recess and further includes a threaded rod adapted to engage the threaded recess so as to move the second member relative to the first member and the threaded rod is made of a harder metal than the threaded recess

Optionally, the threaded recess of the cap is made of a nickel titanium based shape memory alloy (for example a Nitinol alloy) and the threaded rod is made of a stainless steel.

Optionally, the threaded recess of the cap is made of a relatively soft stainless steel such as that sold under the trade mark Nitronic 60. This is high silicon, high manganese, nitrogen strengthened austenitic stainless alloy. The threaded rod can then be made of 455 custom stainless steel.

Optionally, the second member defines a second member longitudinal axis thereof, and a washer system is also included and is coupled to the second member. A drive mechanism is coupled to washer system, such that as the drive mechanism is activated, the washer system rotates about the second member longitudinal axis and expands along the second member longitudinal axis, in which such movement further causes the second member to move relative to the first member along the second member longitudinal axis.

The tool of the invention can be used in a method of assembling a first component of a prosthesis to a second component of the prosthesis for use in joint arthroplasty. The method includes using an assembly tool having a first member and a second member. The second member defines a second member longitudinal axis. The assembly tool also includes a washer system coupled to the second member and a drive mechanism coupled to the washer system. The first component of the prosthesis is inserted onto the second component of the prosthesis. The second member of the assembly tool is secured onto the second component of the prosthesis. The drive mechanism is activated, causing the second member to move relative to the first member along the second member longitudinal axis.

The invention is described below by way of example with reference to the accompanying drawings, in which:
FIG. 1 is a cut-away perspective view of an assembly tool.
FIG. 2 is cut-away view of the assembly tool of FIG. 1 coupled to a modular implant.
FIG. 3 is an enlarged cut-away view of FIG. 2.
FIG. 4 is a perspective view of an assembly tool.
FIG. 5 is a perspective view of the washer assembly of FIG. 1.
FIG. 6 is a perspective view of the washer assembly of FIG. 5 in a rotated position.
FIG. 7 is a cut-away view of a tensile rod assembly of the assembly tool of FIG. 1.
FIG. 7a is a close-up view of a washer system and a pull rod.
FIG. 8 is a flow chart of the method for using an assembly tool.
FIG. 9 is a partial cut-away perspective view of an assembly tool.
FIG. 10 is another perspective view of the assembly tool of FIG. 9.
FIG. 11 is a partially exploded view of the assembly tool of FIG. 9.
FIG. 11a is a close-up view of a portion of the assembly tool of FIG. 11.
FIG. 12 is a cut-away view of a first member of the assembly tool of FIG. 9.
FIG. 13 is a perspective view of a tensile bar and housing of the assembly tool of FIG. 9.

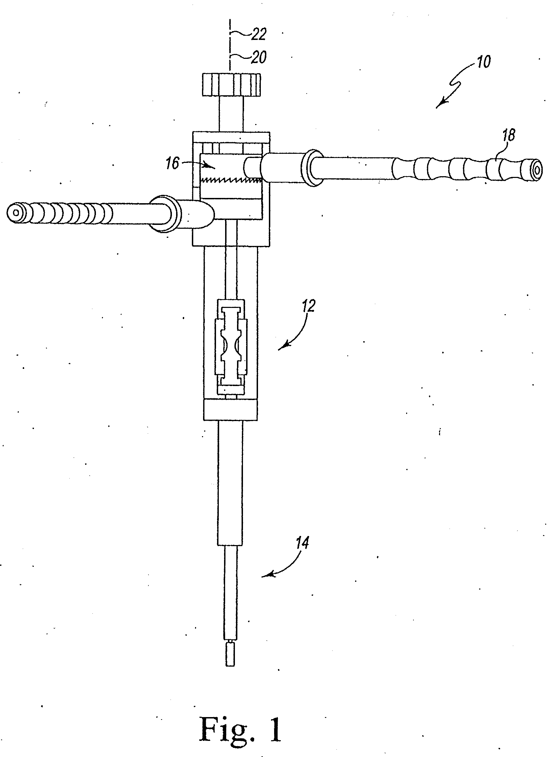

FIGS. 1 to 7 show a tool which does not have all of the features of the invention. They are included in this document to aid understanding of the invention.

FIG. 1 is a perspective view of an assembly tool 10. The assembly tool 10 includes a first member 12 and a second member 14. Coupled to the second member 14 is a washer system 16. A drive mechanism 18 is coupled to the washer system 16. The first member 12 has a first member longitudinal axis 20 and the second member 14 has a second member longitudinal axis 22. In the tool shown in the drawings, the first member longitudinal axis 20 and the second member longitudinal axis 22 are co-incident. The two axes 20, 22 may however be parallel or offset at an angle from one another. As the drive mechanism 18 is activated, it causes the washer system 16 to rotate about the second member longitudinal axis 22. In the tool shown in the drawings, the drive mechanism is a handle that is ratcheted about the second member longitudinal axis 22. The drive mechanism could however be a longitudinal handle, a Hudson connection that connects to a power source, or other known drive mechanism that would cause the washer system 16 to rotate about the second member longitudinal axis 22.

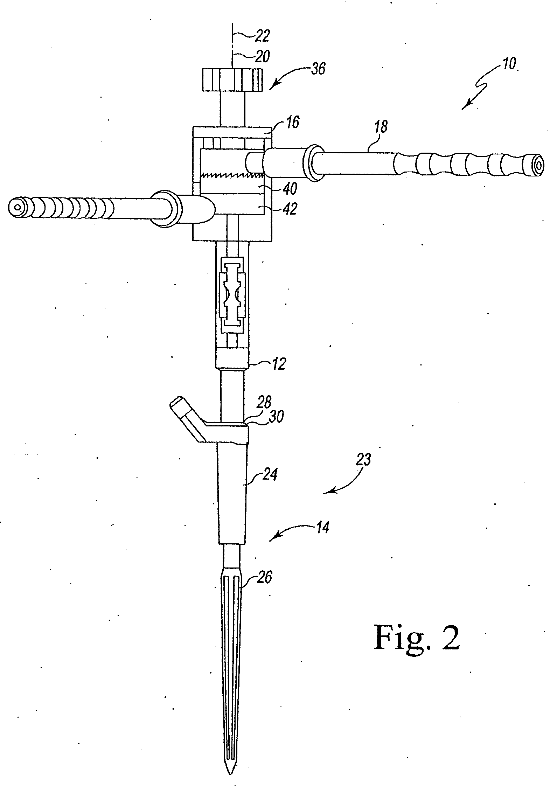

FIG. 2 is a cut-away view of the assembly tool 10 coupled to a modular implant 23. As shown, the modular implant 23 includes a first component 24 (or a proximal or neck component) and a second component 26 (or a distal or stem component). The first member 12 of the assembly tool 10 includes a distal end 28 that abuts a proximal end 30 of the neck component 24. Other connection means may be used. For example, the distal end 28 may include threads that engage a threaded end of the proximal end 30 of the neck component 24. The connection means may also be a retractable button/recess system, a slotted 1-shaped recess and rod system, an undercut, an expandable collet system, or any other known engagement system.

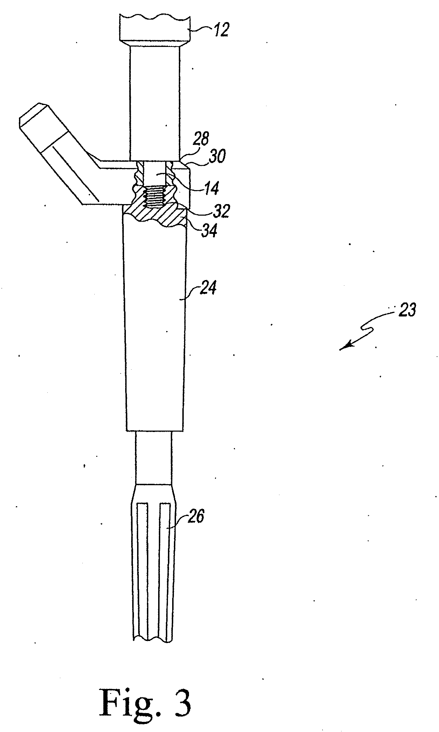

As shown in FIG. 3, a distal end 32 of the second member 14 engages a proximal end 34 of the stem component 26. In tool shown in the drawings, the distal end 32 of the second member 14 is threaded and fits inside a threaded bore of the proximal end 34 of the stem component 26. Alternatively, the distal end 32 of the second member 14 may have the threaded bore and the proximal end 34 of the stem component may be threaded. Other known means of connecting pieces may be used. For example, an expandable collet may be used. Alternatively, the connection means may be a retractable button/recess system, an undercut, a slotted 1-shaped recess and rod system, an expandable collet system, or any other known engagement system.

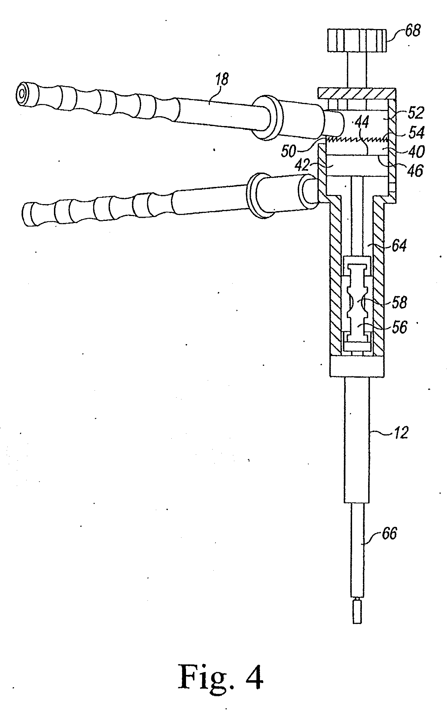

The second member 14 also includes a proximal end 36 (FIG. 2). The proximal end 36 includes a knob 68 (FIG. 4). The knob 68 is coupled to the threaded distal end 32, such that as the knob 68 is rotated about the second member longitudinal axis 22, the threaded distal end 32 is threaded into the threaded bore proximal end 34 of the stem component 26.

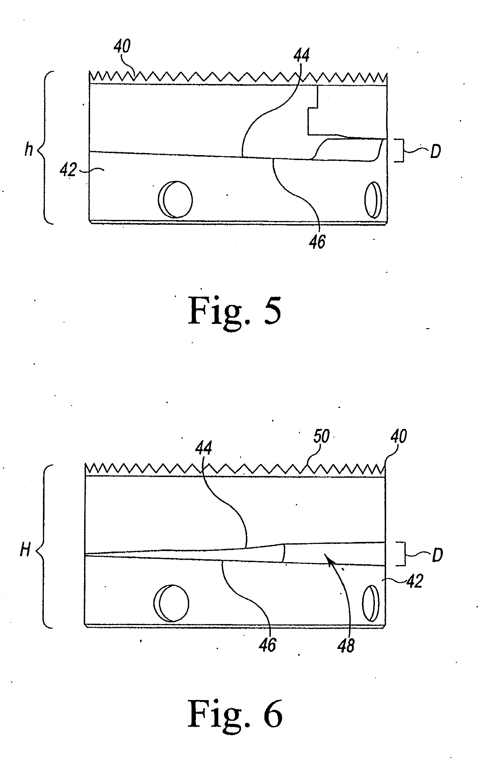

FIGS. 4 to 6 show the washer system 16 which includes a first spiral washer 40 and a second spiral washer 42. The first spiral washer 40 is coupled to the drive mechanism 18 and includes a first spiral ramp 44 and the second spiral washer 42 is coupled to the second member 14 and the first spiral washer 40 and includes a second spiral ramp 46 (FIG. 5). The first spiral ramp 44 abuts the second spiral ramp 46. The washer system 16 has an overall starting height of h. As the first spiral ramp 44 is rotated relative to the second spiral ramp 46, the ramps engage one another, creating a gap 48 between the first spiral washer 40 and the second spiral washer 42. The gap 48 is of a distance D. As shown in FIGS. 5 and 6, the washers 40, 42 begin by being flush against one another. However, as the first spiral washer 40 is rotated, the spiral ramps 44, 46 are rotated enlarging the height of the two washers 40, 42. The distance D of the gap 48 remains the same. In FIG. 6, the overall height of the washer system 16 is now H, which is larger than h. This change in height is generated by the opposing spiral ramps 44, 46 engaging one another, creating a washer system 16 with a variable height.

As shown in FIG. 4, the first washer 40 includes a ratchet end 50 that opposes the spiral ramp 44. The first washer 40 is coupled to a ratchet washer 52. The ratchet washer 52 is connected to the handle 18. One side 54 of the ratchet washer 52 is ratcheted and mates with the ratchet end 50 of the first washer. As the handle 18 is turned, the ratchets on the ratchet washer 52 and first spiral washer 40 engage one another, causing the first spiral washer 40 to rotate. Because the second spiral washer 42 is fixed, the two spiral ramps 44, 46 engage and cause the first spiral washer 40 to become raised (by a height D) relative to the second spiral washer 42.

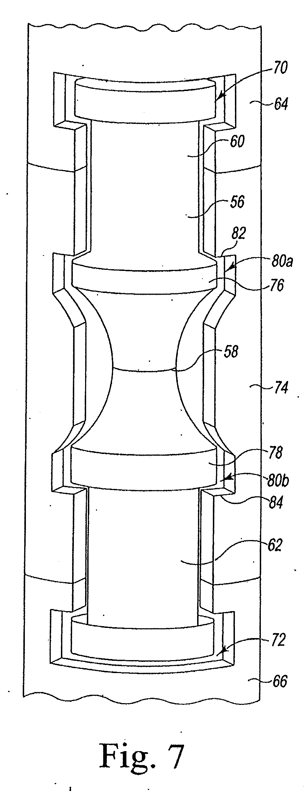

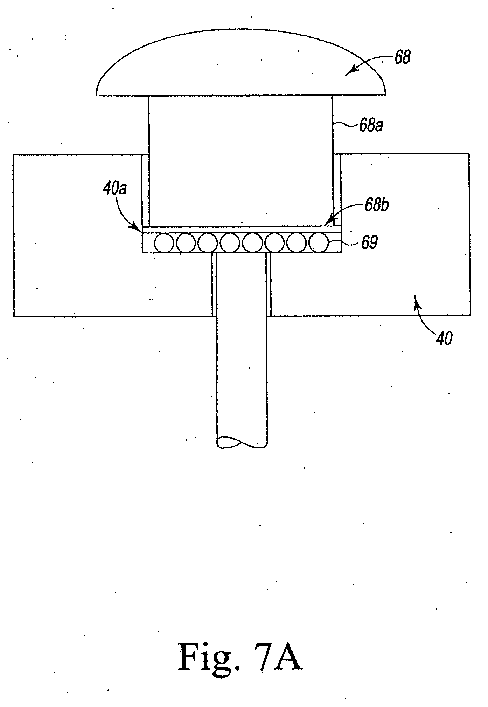

Referring still to FIG. 4 and also to FIG. 7, the second member 14 includes a sacrificial member 56, which in this case is a tensile rod or shear pin. The tensile rod 56 includes an intentional weak spot or breakage point 58. The breakage point 58 can only tolerate up to a specific tension. After that load (or tension) is reached, the breakage point 58 breaks, leaving two separate pieces 60, 62. As shown in FIG. 4, the tensile rod 56 links an upper part 64 of the second member 14 to a lower part 66 of the second member 14. As the handle 18 is turned, and the threaded distal end 32 of the second member 14 is threaded into the threaded bore of the proximal end 34 of the stem component 26, tension is created.

At the top of the upper part 64 of the second member 14, there is the knob 68, as described above. The knob 68 is turned to first thread the threaded end 32 of the second member 14 to the stem component 26.

The knob 68 is coupled to the first spiral washer 40 in any number of known methods. In the instrument shown in FIG. 7a, the knob 68 includes a pull rod 68a having a shoulder 68b. The shoulder 68b is coupled to a counterbore 40a in the first spiral washer 40, such that as the first spiral washer 40 rotates and moves upwards along the longitudinal axis 22, the knob 68 also moves upwards. In tool shown in the drawings, a bearing 69 is located between the shoulder 68b and the counterbore 40a. The bearing 69 illustrated is a rolling bearing and reduces the friction and torsional force felt by the pull rod 68a (and thus the sacrificial member 56). By reducing the frictional and torsional forces felt by the sacrificial member 56, the linear force at which the sacrificial member 56 will break is kept more consistent. Other types of bearings may be used, or no bearing 69 may be used and the shoulder 68b abuts the counterbore 40a directly.

As stated above, as the first spiral washer 40 rotates, and moves up along the longitudinal axis 22, the knob 68 also moves upwards. Because the knob 68 and threaded end 32 are coupled to one another and the threaded end is fixed within the stem component, the movement of the knob 68 creates tension along the second member 14. Once the tension reaches a certain force (or load), the tensile rod 56 will break at the breakage point 58. A loud noise will be heard and the knob 68 will become loose. The tensile rod 56 breaking is important because it signals to the user that enough force has been applied. In tool shown in the drawings, the tensile rod 56 is fixed to break at a predetermined force, for example between about 8900 N and about 11100 N (about 2000 lbf and about 2500 lbf), and preferably at about 10000 N (about 2250 lbf). Optionally, the knob 68 may also be used to disengage the ratchet washer 52 from the ratchet end 50 of the first washer 40.

As shown in FIG. 7, the two halves 60, 62 of the tensile rod 56 are each fitted into slotted openings 70, 72, respectively, of the second member 14. A sleeve 74 fits around the tensile rod 56. As shown, the first half 60 and the second half 62 each include a rib 76, 78 respectively, that extends outwardly. The ribs 76, 78 each fit within a recess 80a, 80b of the sleeve 74. The ribs 76, 78 also engage an edge 82, 84 of the recesses 80a, 80b. Once the tensile rod 56 breaks, both the first and second halves 60, 62 remain contained within the sleeve 74. Even though the first and second halves 60, 62 are no longer connected directly to one another, rotation of one will cause the other to rotate. As the knob 68 is rotated, the slotted opening 70 rotates. This rotation causes the first half 60 of the tensile rod 56 to rotate. When the first half 60 rotates, it engages the edge 82 of the sleeve 74, causing the sleeve 74 to rotate. As the sleeve 74 rotates, the edge 84 engages the second half 62, causing the second half 62 to rotate. The second half 62 rotating engages the slotted opening 72, causing the lower portion 66 of the second member 14 to rotate, disengaging the threaded end 32 from the stem component 26. Alternatively, the two halves 60, 62 of the tensile rod 56 are keyed together, such that even after the halves 60, 62 break, they are still coupled together. Then, when one half rotates, the other half also is forced to rotate.

The tensile rod 56 is held by the second member. It may however be held by the first member. Also, any known containment method may be used. Alternatively, the tensile rod 56 need not be contained.

Optionally, the sacrificial member 56 may not be a tensile rod, but could be a torsional member. Once loads are applied on a longitudinal axis, the torsional member feels rotational force (e.g. a torsional spring). The torsional spring could be weakened so as to break at a certain force. The sacrificial member 56 could also be designed to fail in both axial and torsional directions.

Generally, the assembly tool 10 may be made from stainless steel. Optionally, the tensile rod 56 are made from 440C stainless steel, while all other components are made from 17-4 stainless steel. The assembly tool 10 may also be made of plastic, with only the washer system 16 and the tensile rod 56 being made of stainless steel. Other metals may be used. The tensile rod 56 could be made from plastic, ceramic, or other polymer. The sleeve 74 could also be made of plastic or other polymer. Optionally, the assembly tool 10 may entirely be made of a single composite material. Optionally, the tensile rod 56 could be a small fixture with a shear pin.

Optionally, the distal end 28 of the first member 12 could include dimples that would create impressions on the proximal end of the neck component 24. The impressions would serve as a direct correlation to the force applied to the modular construct, much like those produced by a Rockwell hardness test machine. The spherical dimples on 28 could be positioned (clocked), such that, three impressions would be created in each use, regardless of the instrument-to-implant orientation. The physical size of the dimples would be predetermined, based on the material hardness of the proximal body. Other dimension (other than spherical) dimples could also be used. Alternatively, a number other than three dimples may be used.

Optionally, there may be a biassing mechanism, such as a wave spring or other type of spring, used to keep the ratchet washer 52 engaged with the ratchet end 50 of the first washer 40. Other springs may be used in the device to cause the first washer 40 to spring back after being ratcheted. Optionally, the spring may be a constant force spring.

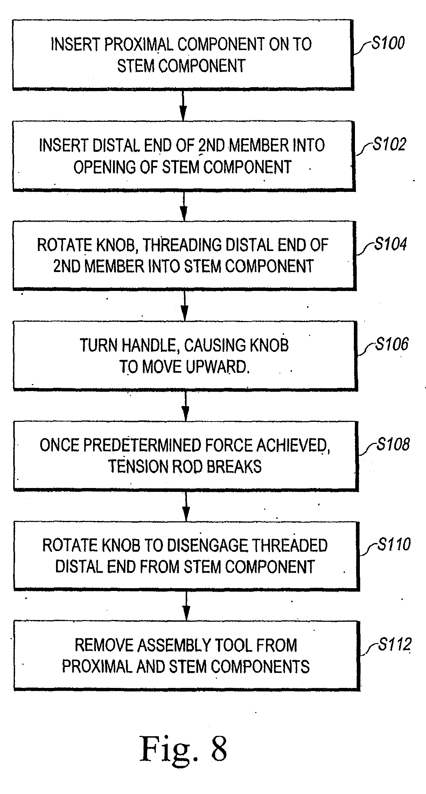

FIG. 8 is a flow chart showing steps in the method of using the assembly tool 10. As shown, at step s100, the proximal component is inserted on to the stem component. Then the distal end of the second member is inserted into the opening of the stem component at step s102. When this is achieved, the distal end of the first member abuts the proximal end of the first component. At step s104, the knob is rotated threading the distal end of the second member into the stem component. The drive mechanism is then turned, causing the knob to move upward (step s106), as described above. Once a predetermined force is applied, the tensile rod breaks, indicating that the proper force has been applied (step s108). At step s110, the knob is rotated to disengage the threaded distal end from the stem component and the assembly tool is removed from the proximal and stem components (step s112).

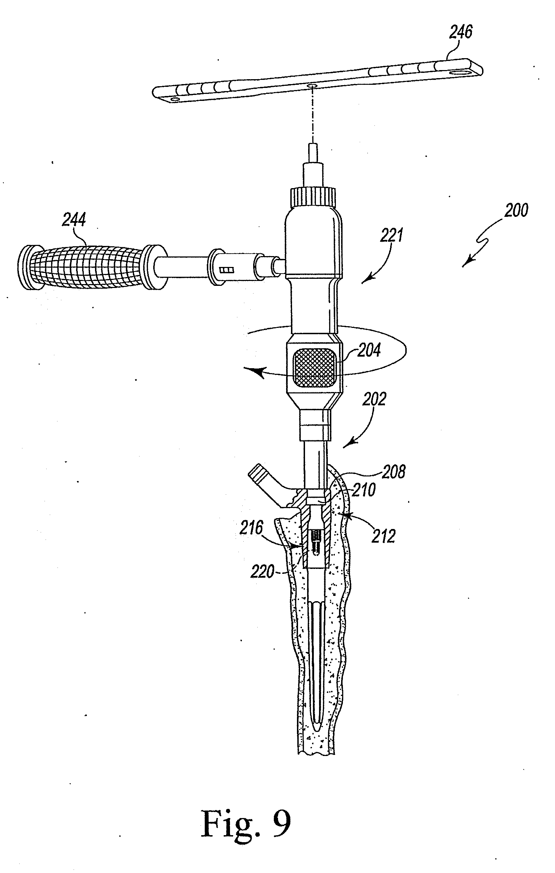

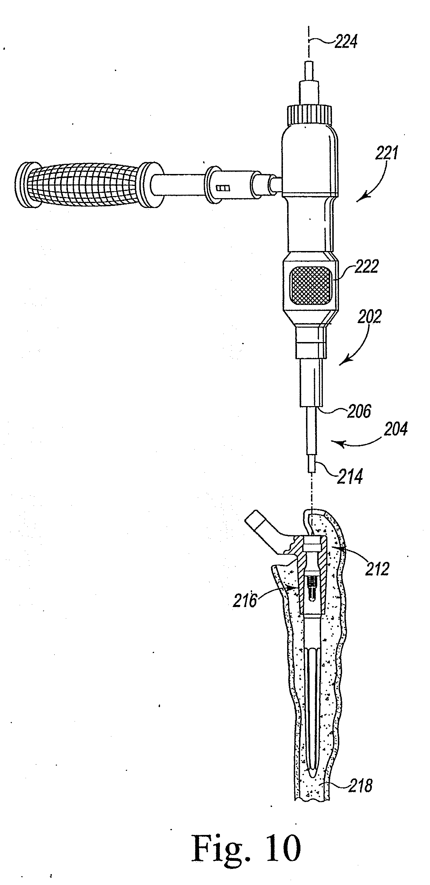

FIGS. 9 and 10 show an assembly tool 200 according to the invention which includes a first member 202 and a second member 204. The first member 202 includes a distal end 206 (FIG. 10) which abuts a proximal end 208 of a neck component 210 of an implant 212. Alternatively, the connection means may be a retractable button/recess system, a slotted 1-shaped recess and rod system, an undercut, an expandable collet system, or any other known engagement system.

A distal end 214 of the second member 204 engages a proximal end 216 of a stem component 218. In this device, the distal end 214 of the second member 204 is threaded and fits inside a threaded bore of the proximal end 220 of the stem component 218. Alternatively, the distal end 214 of the second member 204 may have the threaded bore and the proximal end 216 of the stem component 218 may be threaded. Other known means of connecting pieces may be used. For example, an expandable collet may be used. Alternatively, the connection means may be a retractable button/recess system, an undercut, a slotted 1-shaped recess and rod system, an expandable collet system, or any other known engagement system.

The second member 204 also includes a proximal end 221. The proximal end 221 includes a knob 222. The knob 222 is coupled to the threaded distal end 214, such that as the knob 222 is rotated about a second member longitudinal axis 224, the threaded distal end 214 is threaded into the threaded bore proximal end 220 of the stem component 218.

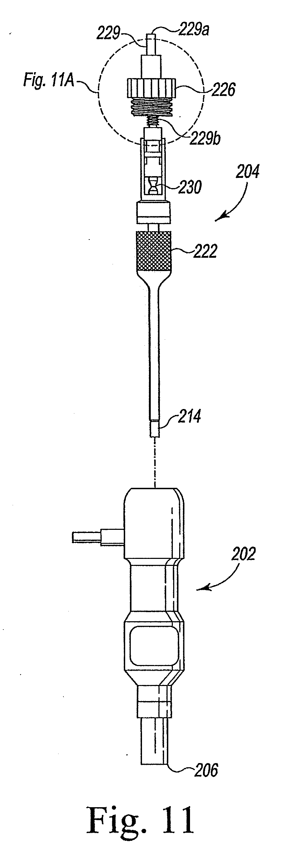

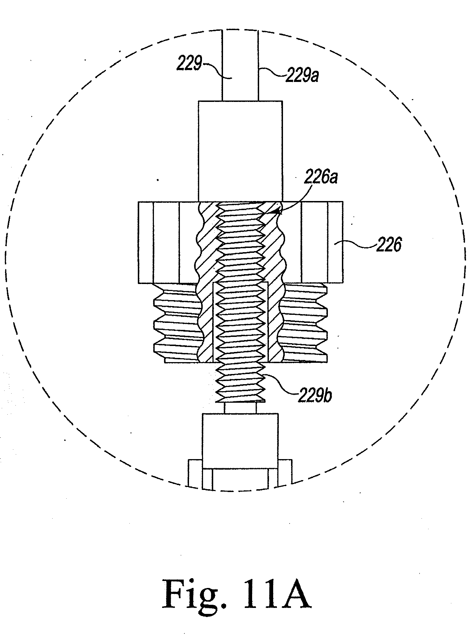

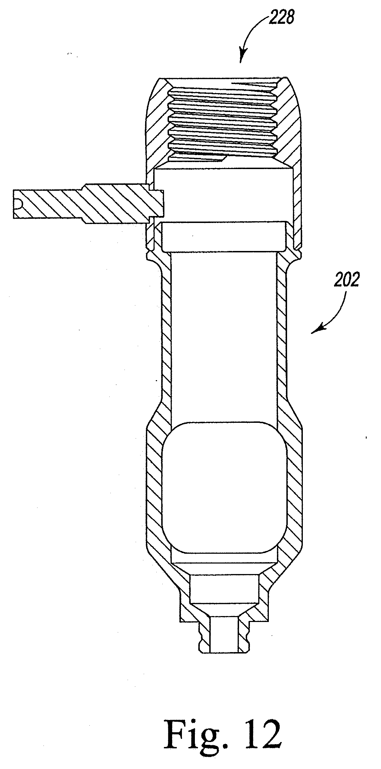

FIG. 11 is an exploded view of the assembly tool 200. As shown, the second member 204 is shown disassembled from the first member 202. The second member 204 includes a threaded cap 226. When assembled, the threaded cap 226 is threaded into a corresponding threaded bore 228 (FIG. 12).

As shown in FIGS. 11 and 11a, a rod 229 having a hex end 229a and a threaded end 229b is shown. The rod 229 is threaded into a threaded recess 226a of the cap 226. The male threads of the threaded end 229b are made of stainless steel, such as 455 custom stainless steel and the cap 226 (including its female threads 226a) are made of a stainless steel, such as Nitronic 60. 455 custom stainless steel is a hard material, while Nitronic 60 is comparably very soft. Thus, when the two are moved relative to one another, there is very little galling and the parts can be cycled a number of times before having to be replaced. The second member 204 also includes a tensile bar 230 that is sized and shaped to break at a predetermined force. This informs the user when the correct force has been reached. Optionally, the force is between about 8900 N and about 11100 N (about 2000 lbf and about 2500 lbf), and preferably at about 10000 N (about 2250 lbf).

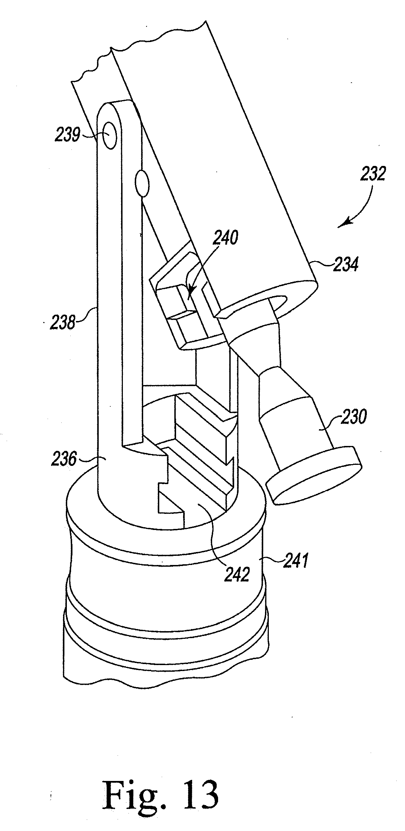

As shown in FIG. 13, the tensile bar 230 is held into place by a housing 232. The housing 232 includes an upper restraint 234 and a lower restraint 236. The upper restraint 234 is pivotally attached to the lower restraint 236 by extension arms 238 on the lower restraint and a pivot pins 239. The upper restraint 234 includes a T-shaped slot 240 that is shaped to engage the tensile bar 230. The opening of the T-shaped slot 240 faces one of the extension arms 238. The lower restraint 236 includes a T-shaped slot 242, with the opening facing perpendicular from the opening of the T-shaped slot 240 of the upper restraint 234. The lower restraint 236 also includes a spring-loaded lower section 241.

In use, the user would pull down on the spring-loaded lower section 241, and pivot the upper restraint 234 into an open position. The user then inserts the tensile bar 230 into the T-shaped slots. The upper restraint 234 is then pivoted back into alignment with the lower restraint 236 and the user releases the spring-loaded lower section 241. The opening of the T-shaped slot 240 of the upper restraint 234 is blocked by one of the extension arms and the opening of the T-shaped slot 242 of the lower restraint 236 is blocked by the released spring-loaded lower section 241.

During use, once the tensile bar 230 breaks, the T-shaped openings 240, 242 keep the respective halves of the tensile bar 230 in place along with the spring-loaded lower section 241 until the user is ready to disassemble the tool 200. Also, as described above with reference to assembly tool 10, the upper and lower restraints 234, 236 are keyed together, so that even after the tensile bar breaks, the upper and lower restraints 234, 236 still move together.

The use of the assembly tool 200 will be described with reference to FIGS. 9 and 10. As shown, the first member 202 includes a stationary handle 244 and a torque handle 246 is inserted onto the second member 204. To use, the assembly tool 200 is inserted into the proximal body 208 such that the distal end 206 of the first member abuts the proximal body 208. The distal end 214 of the second member is inserted into the threaded opening 220 of the stem 218 and the knob 222 of the second member 204 is rotated, causing the threaded distal end 214 of the second member 204 to threadingly engage the threaded opening 220 of the stem 218. The user then rotates the torque handle 246 while holding the handle 244 of the first member 202 stationary. During this use, the rod 229 is rotated relative to the cap 226 and the threaded end 229a is moved relative to the cap 226, thereby moving the threaded distal end 214. The user continues to rotate until an audible sound is heard, indicating the breaking of the tensile bar 230. The user then disengages the tool 200 from the implant by rotating the knob 222 counterclockwise.

## Claims

1. An assembly tool for assembly of a first component of a prosthesis to a second component of the prosthesis for use in joint arthroplasty, the tool comprising:
a first member (202) operably associated with the first component, the first member defining a first member longitudinal axis,
a second member (204) operably associated with the second component, the second member including a tensile bar (230) adapted to break at a predetermined force, and a housing (232) adapted to retain the tensile bar after the tensile bar breaks,
**characterised in that** the housing of the second member includes an upper restraint (234) and a lower restraint (236) which is pivotally attached to the upper restraint, each of the upper and lower restraints having a T-shaped slot (240, 242) formed in it with the opening into the T-shaped slot on the lower restraint being arranged perpendicular to the opening into the T-shaped slot on the upper restraint, the slots being shaped so as to receive respective ends of the tensile bar.

2. The assembly tool of claim 1, in which the lower restraint (236) includes a spring-loaded lower section (241) adapted to contain the tensile bar (230) in the lower restraint.

3. The assembly tool of claim 1, in which the second member includes a cap (226) having a threaded recess (226a) and further includes a threaded rod (229) adapted to engage the threaded recess so as to move the second member relative to the first member and the threaded rod is made of a harder metal than the threaded recess.

## Patentansprüche

1. Montagewerkzeug zur Montage einer ersten Komponente einer Prothese an einer zweiten Komponente der Prothese zur Verwendung in Gelenkarthroplastik, wobei das Werkzeug umfasst:
ein erstes Glied (202), das operabel mit der ersten Komponente assoziiert bzw. verbunden ist, wobei das erste Glied eine Erstes-Glied-Längsachse definiert,
ein zweites Glied (204), das operabel mit der zweiten Komponente assoziiert bzw. verbunden ist, wobei das zweite Glied ein dehnbares Gestänge (230), das angepasst ist, bei einer vorbestimmten Kraft zu brechen, und ein Gehäuse (232) enthält, das angepasst ist, das dehnbare Gestänge zurückzuhalten, nachdem das dehnbare Gestänge bricht,
**dadurch gekennzeichnet, dass** das Gehäuse des zweiten Glieds eine oberen Beschränkung bzw. Zurückhaltung (234) und eine untere Beschränkung bzw. Zurückhaltung (236) enthält, die schwenkbar an der oberen Beschränkung angebracht ist, wobei jede der oberen und unteren Beschränkung einen T-förmigen Schlitz (240, 242) in sich gebildet aufweist, wobei die Öffnung in den T-förmigen Schlitz an der unteren Beschränkung senkrecht zu der Öffnung in den T-förmigen Schlitz an der oberen Beschränkung angeordnet ist, wobei die Schlitze geformt sind, jeweilige Enden des dehnbaren Gestänges aufzunehmen.

2. Montagewerkzeug nach Anspruch 1, bei dem die untere Beschränkung (236) einen federbelasteten bzw. -gespannten unteren Abschnitt (241) enthält, der angepasst ist, das dehnbare Gestänge (230) in der unteren Beschränkung zu halten.

3. Montagewerkzeug nach Anspruch 1, bei dem das zweite Glied eine Kappe (226) mit einer Gewindeaussparung (226a) enthält, und ferner eine Gewindestange (229) enthält, die angepasst ist, in die Gewindeaussparung einzugreifen, um das zweite Glied relativ zu dem ersten Glied zu bewegen, und wobei die Gewindestange aus einem härteren Metall besteht als die Gewindeaussparung.

## Revendications

1. Outil d'assemblage pour assembler un premier composant d'une prothèse à un second composant de la prothèse, destiné à être utilisé pour une arthroplastie articulaire, l'outil comprenant :
un premier élément (202) associé de manière opérationnelle au premier composant, le premier élément définissant un premier axe longitudinal d'élément,
un second élément (204) associé de manière opérationnelle au second composant, le second élément comprenant une barre de traction (230) adaptée pour se rompre à une force prédéterminée, et un boîtier (232) adapté pour retenir la barre de traction après la rupture de la barre de traction,
**caractérisé en ce que** le boîtier du second élément comprend un dispositif de retenue supérieur (234) et un dispositif de retenue inférieur (236) qui est fixé de manière pivotante au dispositif de retenue supérieur, chacun des dispositifs de retenue supérieur et inférieur ayant une fente en forme de T (240, 242) formée dans ce dernier, avec l'ouverture dans la fente en forme de T sur le dispositif de retenue inférieur qui est agencée perpendiculairement à l'ouverture dans la fente en forme de T sur le dispositif de retenue supérieur, les fentes étant formées afin de recevoir les extrémités respectives de la barre de traction.

2. Outil d'assemblage selon la revendication 1, dans lequel le dispositif de retenue inférieur (236) comprend une section inférieure à ressort (241) adaptée pour contenir la barre de traction (230) dans le dispositif de retenue inférieur.

3. Outil d'assemblage selon la revendication 1, dans lequel le second élément comprend un capuchon (226) ayant un évidement fileté (226a) et comprend en outre une tige filetée (229) adaptée pour mettre en prise l'évidement fileté afin de déplacer le second élément par rapport au premier élément et la tige filetée est réalisée avec un métal plus dur que l'évidement fileté.
